(19) Europäisches Patentamt
European Patent Office
Office européen des brevets

(11) **EP 2 537 512 A2**

## (12) EUROPEAN PATENT APPLICATION

(43) Date of publication:
**26.12.2012 Bulletin 2012/52**

(21) Application number: **12164536.0**

(22) Date of filing: **18.04.2012**

(51) Int Cl.:
*A61K 8/34* *(2006.01)*          *A61K 8/36* *(2006.01)*
*A61K 8/365* *(2006.01)*        *A61K 8/368* *(2006.01)*
*A61K 8/37* *(2006.01)*          *A61K 8/46* *(2006.01)*
*A61K 8/49* *(2006.01)*          *A61Q 19/00* *(2006.01)*
*A61Q 19/02* *(2006.01)*        *A61Q 19/08* *(2006.01)*
*A61P 17/12* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **19.04.2011 US 201113089826**

(71) Applicant: **De Paoli Ambrosi, Gianfranco
25010 San Felice del Benaco (BS) (IT)**

(72) Inventor: **De Paoli Ambrosi, Gianfranco
25010 San Felice del Benaco (BS) (IT)**

(74) Representative: **Long, Giorgio et al
Jacobacci & Partners S.p.A.
Via Senato 8
20121 Milano (IT)**

## (54) Method and formulation for chemical peeling

(57)    The present invention relates to a new method to prepare chemical peeling formulation and a new formulation able to increase the efficacy and the tolerability of preparations based on the use of compounds used to carry out chemical peeling.

More particularly, the present invention refers to a composition for chemical peeling, comprising at least one keratolytic agent and dimethyl isosorbide.

The composition may additionally comprise dimethyl sulphone.

EP 2 537 512 A2

**Description**

**FIELD OF THE INVENTION**

The present invention relates to a new method to prepare chemical peeling formulation and a new formulation able to increase the efficacy and the tolerability of preparations based on the use of compounds used to carry out chemical peeling.

**STATE OF THE ART**

This application is a continuation-in-part of our co-pending application No. 10/557,923, filed 03/10/2006.
A very superficial peeling accelerates the natural exfoliation of the corneous layer, whilst a peeling which acts at a much deeper level causes necrosis and inflammation of the epidermis, the papillary dermis or of the reticular dermis.
Chemical peeling produces apparent changes in the skin through three mechanisms of action:

1. The stimulation of cellular turnover through the removal of the dead cells from the corneous layer.
2. The elimination of damaged and degenerated epidermal cells, which will be replaced by normal epidermal cells. This result will be particularly evident in the treatment of actinic keratosis and anomalous pigmentations.
3. The introduction of an inflammatory reaction and the activation of the mediators of inflammation (a mechanism still poorly understood) which activates the production of new collagen fibres and glycosaminoglycans (revitalising mechanisms of the dermis).

Since the peeling agents which operate at deep epidermal levels also carry the risk of complications and undesired outcome, it is indispensable to carry out treatments and therapies which achieve excellent results with the minor risk.
Chemical peeling is particularly recommended in the following cases:

a) Keratosis and cutaneous ageing
b) Dischromia
c) Post - acne scarring
d) Common acne and rosacea
e) Radiodermatitis
f) Stretch marks
g) Seborrheic dermatitis

The various types of chemical peeling can be thus classified as follows:

- very superficial peeling: this type of peeling only removes the superficial corneous layer;
- superficial peeling: this type of peeling causes necrosis of a part or of all of the epidermal layer reaching the basal layer of the epidermis;
- average depth peeling: this type of peeling causes necrosis of the epidermis and part of the papillary dermis;
- deep peeling: this type of peeling causes necrosis of the epidermis, the papillary dermis and can extend to the reticular dermis.

For chemical peeling the following chemical substances are generally used:

1. Retinoic acid
2. 5-Fluorouracile (5-FU)
3. Jessner's Solution
4. Resorcine
5. Salicylic acid
6. Trichloroacetic acid
7. Alpha-hydroxy-acids
8. Alpha-keto-acids (such as, for example, pyruvic acid)
9. Phenol

The depth of peeling depends on numerous factors, such as: i) the type of substance used; ii) the concentration of the substance used, iii) the number of steps with the chosen substance on the same part of the skin, iv) the application technique, v) the preparation of the skin in the pre-treatment phase, vi) the type of cutaneous treatment in the period preceding the peeling, vii) the patients skin type, viii) the area of the skin treated, and ix) the exposure time to the selected chemical agent on the skin.

Considering all these variables, it is easy to understand that any classification relating to the various types of peeling has problems, since with the same substance we can obtain a superficial result on one type of skin, whilst on another type of skin we can obtain a medium or deep peeling. It is therefore not rare to cause even considerable damage to the skin, damage which, due to the variability delineated above, is frequently difficult to foresee.

The problem which lies at the heart of the present invention is therefore that of making available a formulation which allows on the one hand the attainment of an efficacious chemical peeling and which on the other hand minimises the risks of damage to the skin of the treated subject.

Such a problem is solved by a method to prepare a formulation for chemical peeling and by a formulation as delineated in the attached claims.

## DETAILED DESCRIPTION OF THE INVENTION

Figure Description:

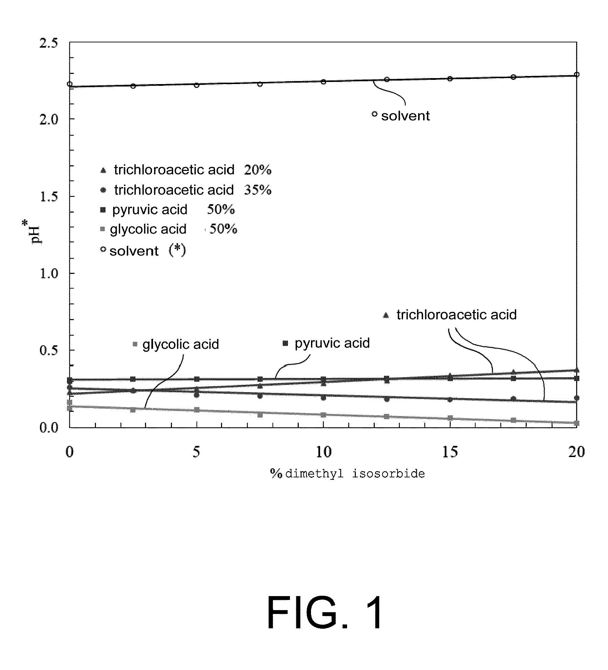

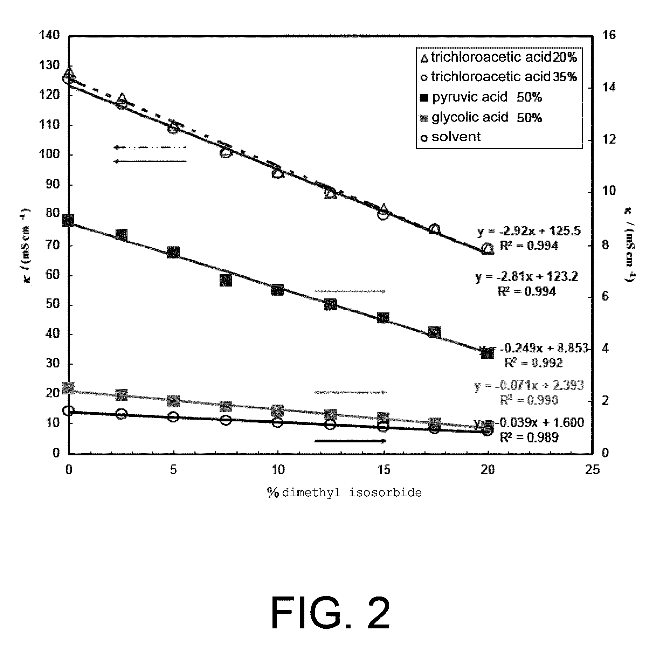

Figure 1. The graph reports the pH as evaluated by using the described experimental method for the indicated series of solutions. pH values are reported as a function of the dimethyl isosorbide % (w/w) in each one of the tested solutions. Figure 2. The graph reports the specific conductivity, as evaluated by using the described experimental method for the indicated series of solutions. Conductivity values are reported as a function of the dimethyl isosorbide % (w/w) in each one of the tested solutions. Conductivity values are reported on the left when referred to trichloroacetic acid, on the right when referred to pyruvic and glycolic acid.

It has been surprisingly found that the combination between the keratolytic agent and dimethyl isosorbide allows the attainment of an improvement in the absorption kinetics of the compound with keratolytic action; this improvement leads to the use of lower quantities of keratolytic agent than those normally used, despite achieving the same or more efficacious and efficient keratolytic action.

The use of lower quantities of keratolytic agent has as a consequence a drastic reduction of the recognisable side effects in the damage to the epidermis and to the dermis.

Peeling is described as a controlled insult at cutaneous level and it is usually determined by the acid nature of the formulation used and by its absorption profile. The action of the peeling solution on the skin can be characterized by two separated phases:

1. catabolic phase: removal of existing cellular and fibrous structures;
2. anabolic phase: replacement with new epidermal cells and with new amorphous and fibrous structures of the dermis.

These two phases can be described according to specific mechanisms of action of chemical, physiological and biochemical nature. The chemical mechanism is related to the ability to cause acid hydrolysis of the peptide bond. It affects the keratin fibres, the protein component of the corneocytes, the desmosomes which join keratinocytes together and on other protein structures of the skin. The chemical mechanism affects mainly the removal of corneocytes and epidermal keratinocytes and this is called desquamation. The physiological mechanism is determined by the differentiation of cells in the basal layer of the epidermis which replace the eliminated keratinocytes during the desquamation phase with the subsequent formation of new stratum corneum through the natural process of keratinization. The biochemical mechanism comprises the release of cytokines and cellular mediators as a consequence to the exposition to an acidic environment. This leads to both a catabolic and an anabolic phase, wherein interleukin-6 induces fibroblasts to increase production of MMPs (Matrix Metallo Proteinases) that results in the digestion of the fibrous structures of the dermis, while interleukin-la induces the fibroblasts to produce new collagen fibers and hyaluronic acid.

Given that chemoexfoliation can be exerted at different levels of the skin structure, depending from the trauma to be specifically treated, the most suitable chemical peeling has to be selected. In particular, said chemical peeling should be able to affect the skin at a very superficial, superficial, medium or at a deep level.

When preparing a formulation to be used for chemical peeling, one important parameter to be considered is the acidity of the formulation itself and, usually, the electrochemical variable used to evaluate the acidity of an aqueous solution is the concentration of free protons. The pKa of the acid to be used as a keratolytic agent is the parameter of choice to select the agent which is suitable for the grade of chemical peeling to be obtained.

Here, we have surprisingly found a method to prepare the chemical peeling formulation of choice for the trauma to be treated.

In particular, we have observed that the chemoexfoliation obtained using a simple aqueous solution of an organic acid is characterized by an important limiting effect: the intensity of the surface trauma is not proportional to the agents ability to renew the epidermis and to remodel the dermis. In fact, in a standard aqueous solution of an acid, the concentration and mobility of the protons are already at their peak. Due to this, when a standard aqueous solution is put in contact with the skin surface, the relationship between superficial trauma and effective chemoexfoliation is skewed towards irritation, with a consequent decrease in the remodelling capacity as the acid penetrates into the deeper skin layers. Until now, in order to reduce the surface trauma, the concentration of free protons was reduced, partially buffering or damping down the solution and thereby increasing the pH values. In this manner, a decreased irritation but proportionately also a reduced chemoexfoliant efficiency were observed. Surprisingly, we have here developed an innovative method to prepare a chemical peeling formulation capable to achieve a more efficient chemoexfoliation. Through this innovative method, the acidity of the formulation is not only evaluated by measuring the concentration of free protons, but also the mobility of these protons, i.e. the speed with which these protons move in an aqueous system. In particular, the mobility of the protons is assessed by evaluating the electrical conductivity of the formulation. When the electrical conductivity of a formulation is comprised in a defined range, a noticeably slowed down protons mobility is obtained, with a resulting more uniform absorption of the acid and an activation *in situ* of the protons in the skin when the acid is absorbed.

The specific conductivity is evaluated as $k$ / ($S\ cm\text{-}1$), wherein said $k$ is defined as follows:

$$\kappa = \sum_{i}^{ioni} z_i c_i u_i F$$

($F$ = Faraday constant = 96485 C[mol e]$^{-1}$)

The specific conductivity, measuring as described above the ability of a solution to conduct electrical current through the migration of the charged species it contains, depends from:

- the concentration $c_i$,
- the charge $z_i$,
- the mobility $u_i$,

of each type of charged particles $i$ in solution.

The present invention refers more specifically to a formulation for chemical peeling comprising a keratolytic agent in combination with dimethyl isosorbide. The dimethyl isosorbide will be present in such a quantity as to obtain an increase in the absorption kinetics of said keratolytic agent, if compared with the use of the keratolytic agent by itself. The increase in absorption kinetics is evaluated as shown below, i.e. by determining the quantity of keratolytic agent able to permeate through an SCE membrane by HPLC.

Preferred keratolytic compounds are selected from the chemical group of saturated and unsaturated monocarboxylic acids, saturated and unsaturated bicarboxylic acids, tricarboxylic acids, alpha hydroxyacids and beta hydroxyacids of monocarboxylic acids, alpha hydroxyacids and beta hydroxyacids of bicarboxylic acids, alpha hydroxyacids and beta hydroxyacids of tricarboxylic acids, ketoacids, alpha ketoacids, beta ketoacids, of the polycarboxylic acids, of the polyhydroxy monocarboxylic acids, of the polyhydroxy bicarboxylic acids, of the polyhydroxy tricarboxylic acids.

Particularly preferred keratolytic agents are selected from the group comprising glycolic acid, tartaric acid, salicylic acid, citric acid, lactic acid, pyruvic acid, gluconic acid, glucuronic acid, malic acid, oxalic acid, malonic acid, succinic acid, acetic acid, phenol, resorcine, retinoic acid, adapalene, trichloroacetic acid, 5-fluoro uracil, azelaic acid. Keratolytic agents comprised within the scope of the present invention are also the salts, esters, possible cis or trans forms, racemic mixtures and/or the relative dextrorotatory or levorotatory forms of the above listed compounds. Such substances can be used singularly or in associations with each other.

According to a particularly preferred embodiment of the present invention, the pharmaceutical and/or cosmetic composition of dimethyl isosorbide with one or more keratolytic agents additionally comprises dimethyl sulphone.

The dimethyl sulphone combined with keratolytic agents is capable of reducing the erythema induced by the agents themselves. According to this embodiment, the reduction of inflammation, irritation and erythema is obtained through the combination of the activity of the dimethyl sulphone with the fact that the quantity of keratolytic agent used to obtain the "peeling" effect is reduced thanks to the action of the dimethyl isosorbide. This latter component, as mentioned previously, increases the kinetics of percutaneous absorption of the keratolytic agent, rendering it more available for the action intended.

A third particularly preferred embodiment is that in which the keratolytic agent and/or the mixed keratolytic agents, combined with dimethyl isosorbide and dimethyl sulphone, are associated with the ester of an acid with keratolytic activity.

When used in association, the dimethyl isosorbide and the keratolytic agent and/or a mixture of keratolytic agents, can each be contained in the composition in a quantity by weight of from 1 to 99%, preferably each in a quantity comprised of between 5 and 40%. More preferably, dimethyl isosorbide and the keratolytic agents will be present in the composition in weight ratios comprised of between 1 : 4 and 4 : 1.

When used in association, the dimethyl isosorbide and the keratolytic agent and/or a mixture of keratolytic agents, associated with dimethyl sulphone can be contained in the compositions in a quantity by weight of from 1 to 99% each, preferably in a quantity comprised of between 5 and 70%. More preferably, dimethyl isosorbide and the keratolytic agent will be present in the composition in a ratio comprised of between 1 : 4 and 4 : 1. The dimethyl sulphone will preferably be present in a quantity comprised of between 2% and 70% by weight, more preferably between 10% and 65%, with respect to the keratolytic agent.

In the compositions of the invention, both based on keratolytic agent/dimethyl isosorbide and keratolytic agent/ isosorbide/dimethyl sulphone, the weight balance up to 100% will be attained by the addition of solvents - such as water (in particular demineralised water), alcohols (such as ethyl alcohol) or glycols (for example, ethylene glycol or propylene glycol) - and/or excipients such as emulsifiers, antioxidants, lipid excipients, sequestrants, preservatives. Such excipients, used in particular for the preparation of emulsions, gels, creams, ointments, etc., are widely known to the expert in the field and will therefore not be described in any further detail.

The method here claimed to prepare a formulation comprising a keratolytic agent and dimethyl isosorbide efficacious in chemical peeling comprises:

> 1) defining the grade of chemical peeling to be obtained: superficial, average or deep peeling;
> 2) selecting one or more keratolytic agent having the pKa in the range suitable for said chemical peeling;
> 3) preparing an aqueous/organic solution of said keratolytic agent, alone or as a mixture of two or more keratolytic agents, and dimethyl isosorbide in water, wherein said keratolytic agent and said dimethyl isosorbide are each present in a concentration able to give rise to a specific conductivity of said solution comprised in the range from 0.005 to 250 mS sec-1;
> wherein said chemical peeling to be obtained is at superficial levels of the skin structure, said specific conductivity varies in the range 0.005-40 mS sec-1 and said pKa vary in the range 2.0-3.0;
> wherein said chemical peeling to be obtained is at average levels of the skin structure, said specific conductivity varies in the range 1.6-6 mS sec-1 and said pKa vary equal or above 3;
> wherein said chemical peeling to be obtained is at deep levels of the skin structure, said specific conductivity varies in the range 40-250 mS sec-1 and said pKa vary in the range 0.5-2.0.

In a preferred embodiment, said water used in step 3) is highly deionised water, preferably, it is MilliQ Millipore grade water. Optionally, dimethyl sulfone should be added to the solution in said step 3). Said specific conductivity is preferably evaluated in a thermostated cells, at a T of about 25°C. Conductivity measurements are carried out in alternating current, using a conductimeter AMEL 160 with a cell (AMEL 192) consisting of a pair of equal platinum foil electrodes, of which works only one of the two sides, this cell was calibrated in advance with a standard 0.1 m KCl solution having, at 25° C, a specific conductivity k = 1.40823 mS cm-1.

The protocol for the preparation of KC1 solutions for calibrating conductimeter provides the use of pure KC1 for analysis dried in an oven (220-240°C for at least 2 hours) and left cool in dryer, and deionised or distilled water, which must be fresh boiled and $CO_2$ free (cooled container in protected tube with soda lime) and must have a conductivity negligible compared to that of the standard solution to be prepared. Note that the standard solutions need to be prepared in molality instead of molarity. In fact, the molality, requesting to weigh both the solute and solvent, is independent from temperature and is inherently more precise. In addition, during the measures the temperature should be monitored, because the specific conductivity depends greatly on the temperature.

An efficacious calibration curve for specific conductivity is obtained by evaluating the specific conductivity on a series of aqueous/organic solutions prepared by dissolving increasing amount of dimethyl isosorbide in water, preferably deionised water.

The evaluation of the specific conductivity has been shown to be effective beyond forecasts, providing reproducible measurements, reliable and clearly and correctly differentiated depending on the strength of the acid. Moreover, a linear trend depending from the percentage of organic co-solvent present was observed, and this is very useful for predictive purpose on the studied formulations. All this features were not obtained considering pH as the only electrochemical parameter. The examples that follows highlights these and other advantages of the here claimed method. Experiments relating to the evaluation of the favourable effect on percutaneous absorption of the keratolytic agent in the presence of dimethyl isosorbide is reported in corroboration of the present invention.

The aim of these experiments has been that of evaluating the *in vitro* percutaneous absorption across isolated human skin, of glycolic acid comprised in a formulation in which the keratolytic agent has been dissolved in water and propylene glycol (solution GC1) and another in which the keratolytic agent has been vehicularised using dimethyl isosorbide (solution GC2).

The experiment has been carried out using a system of Franz cells with a corneous-epidermis membrane layer (SCE membrane), the experimental protocol of which has already been widely described in the literature.

Preparation of the SCE membranes

The preparation of the corneous-epidermis membrane layer (SCE) has been carried out using a technique already described in the literature, using samples of human skin originating from subjects, of ages comprised of between 32 and 45 years, subjected to reductive plastic surgery.

In these skin samples, following separation from the subcutaneous adipose layer and immersion in distilled water at a temperature of 60 °C for a few minutes, the dermis has been separated to obtain the SCE membranes used in this study. The removal of the dermis is made necessary because, in the *in vitro* evaluation of the percutaneous absorption of lipophilic substances, this tissue can be a "dummy" and additional barrier with respect to the *in vivo* cutaneous permeation process. The SCE membranes, thus prepared, have been dried and then placed in an appropriate desiccator. These membranes have then been conserved in sheets of aluminium at a temperature of around 4°C and rehydrated at the time of use, by immersion in distilled water, one hour prior to the start of the permeation experiments. Prior to proceeding to the cutaneous permeation experiments and with the aim of evaluating the integrity of the SCE membranes used, the coefficient of permeability (Kp) of tritiated water has been determined for each sample of SCE membranes, the value of which is a sufficiently indicative parameter of the integrity of said membranes.

Cutaneous permeation experiments

For the evaluation of the degree of *in vitro* percutaneous absorption of glycolic acid from the formulations GC1 and GC2, batteries of six Franz cells (LGA, Berkeley, CA) have been used. Each Franz cell was constituted by a "donor" and a "receptor" between which has been placed the SCE membrane with the corneous layer facing the "donor". The volume of the "receptor" of the cell was 4.7 ml whilst the surface area of the membrane in the "donor" (and therefore the potential cutaneous surface in contact with the product) was $0.75 \ cm^2$.

The "receptor" compartment, stirred and thermostated at a temperature of 35-36 °C, has been fed with an aqueous saline solution of 0.9% (w/v) NaCl.

For the permeation experiment, $200 \ mg/cm^2$ of each formulation GC1 and GC2, containing the glycolic acid, have been initially deposited onto each SCE membrane.

The monitoring of the permeation process has then been carried out by determining, by a suitable HPLC method, the quantity of glycolic acid that has permeated through the corneous-epidermis (SCE) membrane layer over the 24 hour period following the application of the product into the "donor". In order to carry out this experiment samples of SCE membranes originating from six different subjects (n=6) have been used, whilst each single permeation experiment has been performed in duplicate. The results have been expressed as the quantity of permeated glycolic acid, per $cm^2$ of skin, in 24 hours.

HPLC determinations

The determinations of the quantities of glycolic acid, present in the receiver phase of the Franz cell 24 hours after the application of the formulations GC1 and GC2 have been carried out using an appropriate HPLC method reported in the literature.

Results

The results obtained (see Tab. 1) in the studies of the cutaneous permeation of glycolic acid from the formulations GC1 and GC2, demonstrate that the GC2 formulation is able to double (p < 0.01) the quantity of glycolic acid permeated across the SCE membranes with respect to the GC1 formulation.

Tab. 1 - The quantity of glycolic acid (expressed in $\mu$g/cm$^2$) permeated from the formulations GC1 and GC2 across the human skin (SCE) membranes originating from six different subjects (A-F) in 24 hours.

| Subject | CG1 | CG2 |
|---|---|---|
| A | 65.7 | 143.8 |
| B | 82.7 | 169.2 |
| C | 39.4 | 95.3 |
| D | 75.1 | 95.2 |
| E | 112.5 | 197.3 |
| F | 76.1 | 235.4 |
| Mean | 75.2 | 156.0 |
| $\pm$ | 23.7 | 56.1 |

Preparation of calibration solutions:

Step 1) preparation of aqueous/organic solutions (series S).

The solutions listed in Table 2 have been prepared, by using highly deionised water:

Tab. 2

| Series | % (w/w) | | | | | | |
|---|---|---|---|---|---|---|---|
| | Dimethyl isosorbide | Dimethyl sulphone | Ethyl lactate | Ethylic alcohol | Glycol propylenic | Total amount of the addictives A | Total amount of water W |
| S0 | 0 | 0 | 0 | 0 | 0 | 0 | 100 |
| S0' | 0 | 5 | 3 | 2.5 | 2.5 | 13 | 87 |
| S1 | 2.5 | 5 | 3 | 2.5 | 2.5 | 13 | 84.5 |
| S2 | 5 | 5 | 3 | 2.5 | 2.5 | 13 | 82 |
| S3 | 7.5 | 5 | 3 | 2.5 | 2.5 | 13 | 79.5 |
| S4 | 10 | 5 | 3 | 2.5 | 2.5 | 13 | 77 |
| S5 | 12.5 | 5 | 3 | 2.5 | 2.5 | 13 | 74.5 |
| S6 | 15 | 5 | 3 | 2.5 | 2.5 | 13 | 72 |
| S7 | 17.5 | 5 | 3 | 2.5 | 2.5 | 13 | 69.5 |
| S8 | 20 | 5 | 3 | 2.5 | 2.5 | 13 | 67 |

Step 2) solutions of different acids in the solvents prepared in Step 1) are prepared. Table 3 lists 20% trichloroacetic acid solutions prepared (series T):

Tab. 3

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Trichloroacetic acid |
| T0 | 80 | | | | | | | | | | 20 |
| T0' | | 80 | | | | | | | | | 20 |
| T1 | | | 80 | | | | | | | | 20 |

(continued)

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Trichloroacetic acid |
| T2 | | | | 80 | | | | | | | 20 |
| T3 | | | | | 80 | | | | | | 20 |
| T4 | | | | | | 80 | | | | | 20 |
| T5 | | | | | | | 80 | | | | 20 |
| T6 | | | | | | | | 80 | | | 20 |
| T7 | | | | | | | | | 80 | | 20 |
| T8 | | | | | | | | | | 80 | 20 |

Table 4 lists the 35% trichloroacetic acid solutions prepared (series TT):

Tab. 4

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Trichloroacetic acid |
| TT0 | 65 | | | | | | | | | | 35 |
| TT0' | | 65 | | | | | | | | | 35 |
| TT1 | | | 65 | | | | | | | | 35 |
| TT2 | | | | 65 | | | | | | | 35 |
| TT3 | | | | | 65 | | | | | | 35 |
| TT4 | | | | | | 65 | | | | | 35 |
| TT5 | | | | | | | 65 | | | | 35 |
| TT6 | | | | | | | | 65 | | | 35 |
| TT7 | | | | | | | | | 65 | | 35 |
| TT8 | | | | | | | | | | 65 | 35 |

Table 5 lists the 50% pyruvic acid solutions prepared (series P):

Tab. 5

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Pyruvic acid |
| P0 | 50 | | | | | | | | | | 50 |
| P0' | | 50 | | | | | | | | | 50 |
| P1 | | | 50 | | | | | | | | 50 |
| P2 | | | | 50 | | | | | | | 50 |
| P3 | | | | | 50 | | | | | | 50 |
| P4 | | | | | | 50 | | | | | 50 |
| P5 | | | | | | | 50 | | | | 50 |
| P6 | | | | | | | | 50 | | | 50 |

(continued)

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Pyruvic acid |
| P7 | | | | | | | | | 50 | | 50 |
| P8 | | | | | | | | | | 50 | 50 |

Table 6 lists the 50% glycolic acid solutions prepared (series G):

Tab. 6

| Series | % (w/w) | | | | | | | | | | |
|---|---|---|---|---|---|---|---|---|---|---|---|
| | S0 | S0' | S1 | S2 | S3 | S4 | S5 | S6 | S7 | S8 | Glycolic acid |
| G0 | 50 | | | | | | | | | | 50 |
| G0' | | 50 | | | | | | | | | 50 |
| G1 | | | 50 | | | | | | | | 50 |
| G2 | | | | 50 | | | | | | | 50 |
| G3 | | | | | 50 | | | | | | 50 |
| G4 | | | | | | 50 | | | | | 50 |
| G5 | | | | | | | 50 | | | | 50 |
| G6 | | | | | | | | 50 | | | 50 |
| G7 | | | | | | | | | 50 | | 50 |
| G8 | | | | | | | | | | 50 | 50 |

<u>pH evaluation</u>

pH has been evaluated in a thermostated cells at about 25°C. The battery used for pH measurements is a customary one, that is formed by a glass electrode (AMEL 211/SGG/12) combined with a saturated aqueous calomelan reference electrode (AMEL SCE 303/SCG/12, which is checked periodically with respect to the potential with reference to two other electrodes of the same type maintained in saturated KCI). The measuring device was a mVmeter / pH meter AMEL 338, with a resolution of 0.1 mV, which corresponds to $\pm$ 0.0017 pH units. The system was calibrated at 25 °C with four sample solutions distributed on the interval of pH acids, namely: (a) potassium dihydrogen tetraossalate 0.05 m (pH = 1.679), (b) Veibel solution (HC1 0.01 m + KC1 0.09 m, pH = 2.075), (c) commercial buffer pH 4.00 (Merck), (d) commercial buffer pH 7.00 (Merck).
pH has been evaluated on the five series of solutions, namely S, T, TT, P and G. The obtained results are reported in Fig. 1
To note, the measures have a smooth linear flow in all cases and this is in favour of the accuracy of experimental measurements and of the goodness of the protocol followed, given the very narrow range of values. The problem is that said measures are comprised in a very narrow pH range (2/10 of pH only) for all the different acids considered.

<u>Conductivity evaluation</u>

Conductivity has been evaluated in a thermostated cells at about 25°C. Conductivity measurements were carried out in alternating current, using a conductimeter AMEL 160 with a cell (AMEL 192) consisting of a pair of platinum foil electrodes (of which works only one of the two sides), cell previously calibrated with a standard solution of KCI 0.1 m, having at 25 °C a specific conductivity k = 1.40823 mS cm-1.
The obtained results are reported in Fig. 2.
To note, unlike the measures of pH (which all fell together in a small range, and in a different order than the pKa), the conductivity measures are effective, even in these "extreme" conditions, to clearly and correctly monitor the strength of the three different acids; moreover, the conductivity of the solutions shows excellent linear trends in all cases when varying the percentage of co-solvent dimethyl isosorbide. These lines are particularly advantageous for purposes of calibration and / or prediction.

In the following are herein reported some examples of formulations according to the present invention.

EXAMPLES of formulations

Preparation 1

| N° | Description | % (w/w) a |
|----|-------------|-----------|
| 01 | Dimethyl isosorbide | 50.00 |
| 02 | Pyruvic acid | 50.00 |

Method of preparation: mix 01 and 02

Preparation 2

| N° | Description | % (w/w) a |
|----|-------------|-----------|
| 01 | Dimethyl isosorbide | 40.00 |
| 02 | Pyruvic acid | 50.00 |
| 03 | Dimethyl sulphone | 10.00 |

Method of preparation: dissolve 03 in 01, to the solution obtained, mix in 02

Preparation 3

| N° | Description | % (w/w) a |
|----|-------------|-----------|
| 01 | Dimethyl isosorbide | 50.00 |
| 02 | Acetic acid | 50.00 |

Method of preparation: mix 01 and 02

Preparation 4

| N° | Description | % (w/w) a |
|----|-------------|-----------|
| 01 | Dimethyl isosorbide | 40.00 |
| 02 | Acetic acid | 50.00 |
| 03 | Dimethyl sulphone | 10.00 |

Method of preparation: dissolve 03 in 01, to the solution obtained, mix in 02

Preparation 5

| N° | Description | % (w/w) a |
|----|-------------|-----------|
| 01 | Dimethyl isosorbide | 75.00 |
| 02 | Trichloroacetic acid | 25.00 |

Method of preparation: mix 02 in 01

Preparation 6

| N° | Description | % (w/w) a |
|----|-------------|-----------|
| 01 | Dimethyl isosorbide | 75,00 |
| 02 | Trichloroacetic acid | 15,00 |
| 03 | Dimethyl sulphone | 10,00 |

Method of preparation: dissolve 03 in 01, to the solution obtained, mix in 02

Preparation 7

| N° | Description | % (w/w) a |
|----|-------------|-----------|
| 01 | Dimethyl isosorbide | 75.00 |
| 02 | Salicylic acid | 25.00 |

Method of preparation: mix 02 in 01

Preparation 8

| N° | Description | % (w/w) a |
|----|-------------|-----------|
| 01 | Dimethyl isosorbide | 75.00 |
| 02 | Salicylic acid | 15.00 |
| 03 | Dimethyl sulphone | 10.00 |

Method of preparation: dissolve 03 in 01, to the solution obtained, mix in 02

Preparation 9

| N° | Description | % (w/w) a |
|----|-------------|-----------|
| 01 | Dimethyl isosorbide | 20.00 |
| 02 | Tartaric acid | 30.00 |

(continued)

| N° | Description | % (w/w) a |
|----|-------------|-----------|
| 03 | Propylene glycol | 15.00 |
| 04 | Water | 35.00 |

Method of preparation: dissolve 02 in 01, to the solution obtained, mix in 03+04

Preparation 10

| N° | Description | % (w/w) a |
|----|-------------|-----------|
| 01 | Dimethyl isosorbide | 30.00 |
| 02 | Glycolic acid | 60.00 |
| 03 | Water | 10.00 |

Method of preparation: dissolve 02 in 01. Mi the solution obtained with 03

Preparation 11

| N° | Description | % (w/w) a |
|----|-------------|-----------|
| 01 | Dimethyl isosorbide | 30.00 |
| 02 | Glycolic acid | 50.00 |
| 03 | dimethyl sulfone | 5.00 |
| 04 | Water | 15.00 |

Method of preparation: dissolve 02 + 03 in 01. Mix the solution obtained in 02

Preparation 12

| N° | Description | % (w/w) a |
|----|-------------|-----------|
| 01 | Dimethyl isosorbide | 50.00 |
| 02 | Resorcine | 10.00 |
| 03 | Salicylic acid | 20.00 |
| 04 | Ethyl alcohol | 20.00 |

Method of preparation: dissolve 02 + 03 in 04; to the solution obtained mix in 01

Preparation 13

| N° | Description | % (w/w) a |
|---|---|---|
| 01 | Dimethyl isosorbide | 60.00 |
| 02 | Resorcine | 10.00 |
| 03 | Salicylic acid | 20.00 |
| 05 | Ethyl alcohol | 10.00 |

Method of preparation: dissolve 02 + 03 in 05; to the solution obtained mix in 01

Preparation 14

| N° | Description | % (w/w) a |
|---|---|---|
| 01 | Dimethyl isosorbide | 50.00 |
| 02 | Retinoic acid | 1.00 |
| 03 | Salicylic acid | 20.00 |
| 04 | Dimethyl sulphone | 20.00 |
| 05 | Ethyl alcohol | 9.00 |

Method of preparation: mix 01 + 05; dissolve 02 + 03 + 04 in the solution obtained

Preparation 15

| N° | Description | % (w/w) a |
|---|---|---|
| 01 | Dimethyl isosorbide | 20.00 |
| 02 | Glycolic acid | 50.00 |
| 03 | Dimethyl sulphone | 10.00 |
| 04 | Ethyl lactate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

Preparation 16

| N° | Description | % (w/w) a |
|---|---|---|
| 01 | Dimethyl isosorbide | 30.00 |
| 02 | Lactic acid | 40.00 |
| 03 | Dimethyl sulphone | 10.00 |
| 04 | Ethyl lactate | 10.00 |

(continued)

| N° | Description | % (w/w) a |
|---|---|---|
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

Preparation 17

| N° | Description | % (w/w) a |
|---|---|---|
| 01 | Dimethyl isosorbide | 50.00 |
| 02 | Trichloroacetic acid | 20.00 |
| 03 | Dimethyl sulphone | 10.00 |
| 04 | Ethyl pyruvate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

Preparation 18

| N° | Description | % (w/w) a |
|---|---|---|
| 01 | Dimethyl isosorbide | 50.00 |
| 02 | Salicylic acid | 20.00 |
| 03 | Dimethyl sulphone | 10.00 |
| 04 | Ethyl pyruvate | 10.00 |
| 05 | Ethyl alcohol 95° | 5.00 |
| 06 | Propylene glycol | 1.00 |
| 07 | Demineralised water | 4.00 |

Method of preparation: dissolve 03 + 04 in 01: to the solution obtained mix in 02 + 05 + 06 +07;

Preparation 19

| N° | Description | % (w/w) a |
|---|---|---|
|  | PHASE A |  |
| 01 | Steareth 2 | 3.00 |

(continued)

| N° | Description | % (w/w) a |
|---|---|---|
| | PHASE A | |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |
| 08 | Propylene glycol | 2.00 |
| 09 | Pyruvic acid | 10.00 |
| 10 | Demineralised water | 10.00 |
| | PHASE C | |
| 11 | Dimethyl sulphone | 10.00 |
| 12 | Propylene glycol | 2.00 |
| 13 | Disodium EDTA | 0.07 |
| 14 | Glycerol | 5.00 |
| 15 | Phenoxyethanol | 1.00 |
| 16 | Methyl paraben | 0.10 |
| 17 | Ethyl paraben | 0.10 |
| 18 | Propyl paraben | 0.10 |
| 19 | Demineralised water qba | 100 |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

Preparation 20

| N° | Description | % (w/w) a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl lactate | 5.00 |

(continued)

| | | |
|---|---|---|
| | PHASE B | |
| 08 | Propylene glycol | 2.00 |
| 09 | Tartaric acid | 15.00 |
| 10 | Demineralised water | 10.00 |
| | PHASE C | |
| 11 | Dimethyl sulphone | 10.00 |
| 12 | Propylene glycol | 2.00 |
| 13 | Disodium EDTA | 0.07 |
| 14 | Glycerol | 5.00 |
| 15 | Phenoxyethanol | 1.00 |
| 16 | Methyl paraben | 0.10 |
| 17 | Ethyl paraben | 0.10 |
| 18 | Propyl paraben | 0.10 |
| 19 | Demineralised water qba | 100 |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

Preparation 21

| N° | Description | % (w/w) a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Zinc oxide oily solution 50% | 20.00 |
| | PHASE B | |
| 08 | Propylene glycol | 2.00 |
| 09 | Lactic acid | 10.00 |
| 10 | Demineralised water | 10.00 |
| | PHASE C | |
| 11 | Dimethyl sulphone | 10.00 |
| 12 | Propylene glycol | 2.00 |

(continued)

|    | PHASE C                  |      |
|----|--------------------------|------|
| 13 | Disodium EDTA            | 0.07 |
| 14 | Glycerol                 | 5.00 |
| 15 | Phenoxyethanol           | 1.00 |
| 16 | Methyl paraben           | 0.10 |
| 17 | Ethyl paraben            | 0.10 |
| 18 | Propyl paraben           | 0.10 |
| 19 | Demineralised water qba  | 100  |

Method of preparation: heat PHASE A) to 75°C; heat PHASE C to +75°C; combine PHASE A with PHASE C with stirring homogenising the solution; cool to +45°C; then combine with PHASE B still with stirring and cool to 25°C.

In the present invention, the mixture of dimethyl isosorbide, associated with a keratolytic agent and/or a mixture of keratolytic agents, together with dimethyl sulphone, can be combined with esters of the keratolytic agents, preferably the ethyl esters. The use of the keratolytic agent esters, singularly and/or in association, is justified by the fact that, once absorbed into the cuteous, these are hydrolysed liberating the acid and alcohol. The acid form will therefore be able to continue the keratolytic action, in a less intense form but with a more protracted effect over time. Examples of the esters of the keratolytic agents are ethyl pyruvate, ethyl glycolate, triethyl citrate, ethyl resorcinate, the ethyl ester of retinoic acid, ethyl salicylate, methyl salicylate, ethyl malnate, ethyl acetate, ethyl tartrate.

A further subject of the present invention is a formulation for chemical peeling comprising of one or more keratolytic agents, preferably selected from the above described group, together with a keratolytic agent ester. The ester of the keratolytic agent will preferably be selected from the above listed group and can be the ester of the same keratolytic agent used in acid form or the ester of a different keratolytic agent. Such keratolytic agent esters will be present in the composition in a quantity preferably comprised of between 3% and 60% by weight, more preferably in a quantity comprised of between 15% and 50% by weight, with respect to the keratolytic agent (or to the mixture of keratolytic agents).

In place of the keratolytic agent esters, another derivative or pro-drug can however be used, in the same proportions indicated above, which is capable of liberating after administration under biological conditions, the keratolytic agent in the treatment site.

It is evident that the combination of one or more keratolytic agents and their derivative or pro-drug as defined above can also be applied to compositions in which the dimethyl isosorbide or the dimethyl sulphone are not present. One will in fact obtain, in any case, the desired effect of achieving a more prolonged over time and at the same time less acute chemical peeling, with the consequent reduction of the irritant phenomena caused by intense and acute treatment.

**Claims**

1. A method to prepare a formulation comprising at least one keratolytic agent and dimethyl isosorbide efficacious in chemical peeling comprising:

    1) defining the grade of chemical peeling to be obtained: superficial, average or deep peeling;
    2) selecting one or more keratolytic agent having the pKa in the range suitable for said chemical peeling;
    3) preparing an aqueous/organic solution of said keratolytic agent, alone or as a mixture of two or more keratolytic agents, and dimethyl isosorbide in water, wherein said keratolytic agent and said dimethyl isosorbide are each present in a concentration able to give rise to a specific conductivity of said solution comprised in the range from 0.005 to 250 mS sec-1;
    wherein said chemical peeling to be obtained is at superficial levels of the skin structure, said specific conductivity varies in the range 0.005-40 mS sec-1 and said pKa vary in the range 2.0-3.0;
    wherein said chemical peeling to be obtained is at average levels of the skin structure, said specific conductivity varies in the range 1.6-6 mS sec-1 and said pKa vary equal or above 3;
    wherein said chemical peeling to be obtained is at deep levels of the skin structure, said specific conductivity varies in the range 40-250 mS sec-1 and said pKa vary in the range 0.5-2.0.

2. A method according to claim 1, wherein said water used in step 3) is highly deionised water, preferably it is MilliQ Millipore grade water.

3. A method according to claim 1, wherein additionally dimethyl sulphone is added to the solution in said step 3).

4. A method according to any of the claims from 1 to 3, wherein said specific conductivity evaluation is performed in a thermostated cells, at a T of about 25°C.

5. A chemical peeling composition, comprising at least one keratolytic agent and dimethyl isosorbide, prepared by the method according to one of the claims from 1 to 4, having a specific conductivity comprised in the range from 0.005 to 250 mS sec-1

6. A chemical peeling composition, comprising at least one keratolytic agent and dimethyl isosorbide, wherein said keratolytic agent, alone or as a mixture of two or more keratolytic agents, and said dimethyl isosorbide are contained in a weight ratio comprised of between 1 : 4 and 4 : 1.

7. The composition according to claim 6, wherein said keratolytic agent, alone or as a mixture of two or more keratolytic agents, and said dimethyl isosorbide are each contained in a quantity of between 1% and 99% by weight.

8. The composition according to claim 7, wherein said keratolytic agent, alone or as a mixture of two or more keratolytic agents, and said dimethyl isosorbide are each contained in a quantity comprised of between 5% and 40%.

9. A chemical peeling composition, comprising at least one keratolytic agent and dimethyl isosorbide, wherein said formulation has a specific conductivity in the range from 0.005 to 40 mS sec-1 and a pKa in the range 2.0-3.0.

10. A chemical peeling composition, comprising at least one keratolytic agent and dimethyl isosorbide, wherein said formulation has a specific conductivity in the range from 1.6 to 6 mS sec-1 and a pKa vary equal or above 3.

11. A chemical peeling composition, comprising at least one keratolytic agent and dimethyl isosorbide, wherein said formulation has a specific conductivity in the range from 40 to 250 mS sec-1 and a pKa in the range 0.5-2.0.

12. The composition according to anyone of the claims from 5 to 11, wherein said at least one keratolytic agent is selected from saturated and unsaturated monocarboxylic acids, saturated and unsaturated bicarboxylic acids, tricarboxylic acids, alpha hydroxyacids and beta hydroxyacids of monocarboxylic acids, alpha hydroxyacids and beta hydroxyacids of bicarboxylic acids, alpha hydroxyacids and beta hydroxyacids of tricarboxylic acids, ketoacids, alpha ketoacids, beta ketoacids, polycarboxylic acids, polyhydroxy monocarboxylic acids, polyhydroxy bicarboxylic acids, polyhydroxy tricarboxylic acids, salts, esters, possible cis or trans forms, racemic mixtures and/or relative dextrorotatory or levorotatory forms thereof. The composition according to claim 5, wherein said at least one keratolytic agent is selected from glycolic acid, tartaric acid, salicylic acid, citric acid, lactic acid, pyruvic acid, gluconic acid, glucuronic acid, malic acid, oxalic acid, malonic acid, succinic acid, acetic acid, phenol, resorcine, retinoic acid, adapalene, trichloroacetic acid, 5-fluoro uracil, azelaic acid.

13. The composition according to anyone of the claims from 5 to 11, further comprising cosmetically or pharmaceutically acceptable solvents and/or cosmetically or pharmaceutically acceptable excipients.

14. The composition according to claim 13, wherein said solvents are selected from water, alcohols or glycols and said excipients are selected from emulsifiers, antioxidants, lipid excipients, sequestrants, preservatives.

15. The composition according to anyone of the claims from 5 to 14, said composition further comprising dimethyl sulphone.

16. The composition according to claim 15, wherein said dimethyl sulphone is contained in a quantity comprised of between 2% and 70% by weight with respect to the keratolytic agent.

17. The composition according to claim 15, wherein said dimethyl sulphone is contained in a quantity comprised of between 10% and 65% by weight with respect to the keratolytic agent.

18. A composition for chemical peeling, comprising trichloroacetic acid and dimethyl isosorbide in a weight ratio between 1:4 and 4:1.

**19.** A composition according to claim 18, further comprising dimethyl sulphone.

**20.** Use of the composition according to claim 9 in chemical peeling at superficial levels of the skin structure.

**21.** Use of the composition according to claim 10 in chemical peeling at average levels of the skin structure.

**22.** Use of the composition according to claim 11 in chemical peeling at deep levels of the skin structure.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10557923 B